# EUROPEAN PATENT SPECIFICATION

(11) **EP 1 863 819 B1**
(45) Date of publication and mention of the grant of the patent: **12.01.2011**
(21) Application number: 06708216.4
(22) Date of filing: 13.02.2006
(51) Int. Cl.: C07D 487/08, C07D 471/08, A61K 31/551, A61P 25/28

(54) **DIAZABICYCLIC ARYL DERIVATIVES AND THEIR USE AS CHINOLINERGIC LIGANDS AT THE NICOTINIC ACETYLCHOLINE RECEPTORS**
DIAZABICYCLISCHE ARYLDERIVATE UND IHRE VERWENDUNG ALS CHINOLINERGISCHE LIGANDEN AN NIKOTIN-ACETYLCHOLIN-REZEPTOREN
DERIVES ARYLES DIAZABICYCLIQUES ET LEUR UTILISATION COMME LIGANDS CHINOLINERGIQUES DES RECEPTEURS NICOTINIQUES ACETYLCHOLINES

(30) Priority: 16.02.2005 DK 200500230; 17.02.2005 US 653491 P; 06.12.2005 DK 200501723
(43) Date of publication of application: 12.12.2007
(73) Proprietor: NeuroSearch A/S, 2750 Ballerup (DK)
(72) Inventor: PETERS, Dan, DK-2750 Ballerup (DK); TIMMERMANN, Daniel, B., DK-2750 Ballerup (DK); OLSEN, Gunnar, M., DK-2750 Ballerup (DK); NIELSEN, Elsebet, Østergaard, DK-2750 Ballerup (DK); DYHRING, Tino, DK-2750 Ballerup (DK)
(86) International application number: PCT/EP2006/050874
(87) International publication number: WO 2006/087306

(56) References cited:
- WO-A-02/02564
- WO-A-03/094831

## Description

### TECHNICAL FIELD

This invention relates to novel diazabicyclic aryl derivatives, which are found to be cholinergic ligands at the nicotinic acetylcholine receptors and modulators of the monoamine receptors and transporters. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exert its biological effect via two types of cholinergic receptors, the muscarinic Acetyl Choline Receptors (mAChR) and the nicotinic Acetyl Choline Receptors (nAChR).

As it is well established that muscarinic acetylcholine receptors dominate quantitatively over nicotinic acetylcholine receptors in the brain area important to memory and cognition, and much research aimed at the development of agents for the treatment of memory related disorders have focused on the synthesis of muscarinic acetylcholine receptor modulators.

Recently, however, an interest in the development of nAChR modulators has emerged. Several diseases are associated with degeneration of the cholinergic system i.e. senile dementia of the Alzheimer type, vascular dementia and cognitive impairment due to the organic brain damage disease related directly to alcoholism. Indeed several CNS disorders can be attributed to a cholinergic deficiency, a dopaminergic deficiency, an adrenergic deficiency or a serotonergic deficiency.

### SUMMARY OF THE INVENTION

The present invention is devoted to the provision novel modulators of the nicotinic and/or of the monoamine receptors, which modulators are useful for the treatment of diseases or disorders related to the cholinergic receptors, and in particular the nicotinic acetylcholine receptor (nAChR), the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides novel diazabicyclic aryl derivatives represented by Formula I 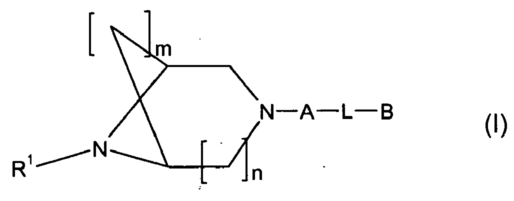 any of its enantiomers or any mixture of its enantiomers, or a pharmaceutically acceptable salt thereof, wherein
m is 2, and n is 1;
R¹ represents hydrogen or C₁₋₆-alkyl ;
A represents a pyridazinyl group;
B represents a phenyl group, which phenyl may optionally be substituted with amino or -NH(CO)R'; wherein R' represents C₁₋₆-alkyl ; and
L represents -C≡C-.

In its second aspect the invention provides pharmaceutical compositions comprising a therapeutically effective amount of the diazabicyclic aryl derivative of the invention, or a pharmaceutically acceptable addition salt thereof, or a prodrug thereof, together with at least one pharmaceutically acceptable carrier or diluent.

In a further aspect the invention relates to the use of the diazabicyclic aryl derivative of the invention, or a pharmaceutically acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

Other objects of the invention will be apparent to the person skilled in the art from the following detailed description and examples.

### DETAILED DISCLOSURE OF THE INVENTION

### Diazabicyclic Aryl Derivatives

In a first aspect novel diazabicyclic aryl derivatives are provided. The diazabicyclic aryl derivatives of the invention may be represented by the general Formula I 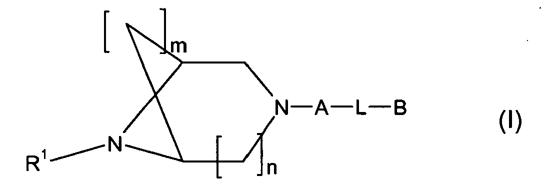 any of its enantiomers or any mixture of its enantiomers, or a pharmaceutically acceptable salt thereof, wherein
m is 2, and n is 1;
R¹ represents hydrogen or C₁₋₆-alkyl;
A represents a pyridazinyl group;
B represents a phenyl group, optionally substituted with amino or -NH(CO)R'; wherein R' represents C₁₋₆-alkyl; and
L represents -C≡C-.

In its most preferred embodiment the diazabicyclic aryl derivative of the invention is
8-Methyl-3-(6-phenylethynyl-pyridazin-3-yl)-3,8-diaza-bicyclo[3.2.1]octane;
N-{4-[6-(8-Methyl-3,8-diaza-bicyclo[3.2.1]oct-3-yl)-pyridazin-3-ylethynyl]-phenyl}-acetamide;
4-[6-(8-Methyl-3,8-diaza-bicyclo[3.2.1]oct-3-yl)-pyridazin-3-ylethynyl]-phenylamine;
N-{4-[6-(8-Methyl-3,8-diaza-bicyclo[3.2.1]oct-3-yl)-pyridazin-3-ylethynyl]-phenyl}-propionamide;
N-{4-[6-(8-Methyl-3,8-diaza-bicyclo[3.2.1]oct-3-yl)-pyridazin-3-ylethynyl]-phenyl}-isobutyramide;
   or
8,8-Dimethyl-3-(6-phenylethynyl-pyridazin-3-yl)-3,8-diaza-bicyclo[3.2.1]octane onium iodide salt;
or an enantiomers or a mixture of its enantiomers, or a pharmaceutically acceptable salt thereof.

### Pharmaceutically Acceptable Salts

The diazabicyclic aryl derivative of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

### Steric Isomers

The chemical compounds of the present invention may exist in (+) and (-) forms as well as in racemic forms. The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or I- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials.

### Methods of Producing Diazabicyclic Aryl Derivatives

The diazabicyclic aryl derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention is devoted to the provision novel ligands and modulators of the nicotinic receptors, which ligands and modulators are useful for the treatment of diseases or disorders related to the cholinergic receptors, and in particular the nicotinic acetylcholine receptor (nAChR). Preferred compounds of the invention show a pronounced nicotinic acetylcholine α7 receptor subtype selectivity.

Due to their pharmacological profile the compounds of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and withdrawal symptoms caused by the termination of abuse of chemical substances.

In a preferred embodiment the compounds of the present invention may be useful for the treatment, prevention or alleviation of a cognitive disorder, learning deficit, memory deficits and dysfunction, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, Amyotrophic Lateral Sclerosis, anxiety, non-0CD anxiety disorders, convulsive disorders, epilepsy, neurodegenerative disorders, transient anoxia, induced neuro-degeneration, neuropathy, diabetic neuropathy, periferic dyslexia, tardive dyskinesia, hyperkinesia, mild, pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Chron's disease, inflammatory bowel disease, ulcerative colitis, diarrhoea, or withdrawal symptoms caused by termination of use of addictive substances, including nicotine containing products such as tobacco, opioids such as heroin, cocaine and morphine, benzodiazepines and benzodiazepine-like drugs, and alcohol.

In a more preferred embodiment the compounds of the invention may be useful for the treatment, prevention or alleviation of pain, mild or moderate or even severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury.

In an even more preferred embodiment the compounds of the invention may be useful for the treatment, prevention or alleviation of diseases, disorders or conditions associated with smooth muscle contractions, convulsive disorders, angina pectoris, premature labour, convulsions, diarrhoea, asthma, epilepsy, tardive dyskinesia, hyperkinesia, premature ejaculation, or erectile difficulty.

In a still more preferred embodiment the compounds of the invention may be useful for the treatment, prevention or alleviation of a neurodegenerative disorder, transient anoxia, or induced neuro-degeneration.

In a yet more preferred embodiment the compounds of the invention may be useful for the treatment, prevention or alleviation of an inflammatory disorder, inflammatory skin disorder, acne, rosacea, Chron's disease, inflammatory bowel disease, ulcerative colitis, or diarrhoea.

In a further preferred embodiment the compounds of the invention may be useful for the treatment, prevention or alleviation of diabetic neuropathy, schizophrenia, cognitive or attentional deficits related to schizophrenia, or depression.

Finally the compounds of the invention may be useful for the treatment of withdrawal symptoms caused by termination of use of addictive substances. Such addictive substances include nicotine containing products such as tobacco, opioids such as heroin, cocaine and morphine, benzodiazepines, benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

It is at present contemplated that a suitable dosage of the active pharmaceutical ingredient (API) is within the range of from about 0.1 to about 1000 mg API per day, more preferred of from about 10 to about 500 mg API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

Preferred compounds of the invention show a biological activity in the sub-micromolar and micromolar range, i.e. of from below 1 to about 100 µM.

### Labelled Compounds

The compounds of the invention may be used in their labelled or unlabelled form. In the context of this invention the labelled compound has one or more atoms replaced by an atom having an atomic mass or mass number different from the atomic mass or mass number usually found in nature. The labelling will allow easy quantitative detection of said compound.

The labelled compounds of the invention may be useful as diagnostic tools, radio tracers, or monitoring agents in various diagnostic methods, and for *in vivo* receptor imaging.

The labelled isomer of the invention preferably contains at least one radionuclide as a label. Positron emitting radionuclides are all candidates for usage. In the context of this invention the radionuclide is preferably selected from ²H (deuterium), ³H (tritium), ¹³C, ¹⁴C, ¹³¹I, ¹²⁵I, ¹²³I, and ¹⁸F.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), or combinations thereof.

### Pharmaceutical Compositions

In another aspect the invention provides novel pharmaceutical compositions comprising a therapeutically effective amount of diazabicyclic aryl derivative of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the diazabicyclic aryl derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by any skilled person by use of standard methods and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depend on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### EXAMPLES

The invention is further illustrated with reference to the following examples, which are not intended to be in any way limiting to the scope of the invention as claimed.

### Example 1

### Preparatory Example

All reactions involving air sensitive reagents or intermediates were performed under nitrogen and in anhydrous solvents. Magnesium sulphate was used as drying agent in the workup-procedures and solvents were evaporated under reduced pressure.

### Diethyl cis-1-methylpyrrolidine-2,5-dicarboxylate (Intermediate compound)

Diethyl *mezo*-2,5-dibromoadipate (101.7 g; 0.283 mol) was dissolved by heating under argon in THF (400 ml) and then cooled to 0°C. To the obtained solution a pre-cooled solution of methylamine (27.3 g; 0.88 mol) in THF (150 ml) was added and the mixture was stirred at room temperature for 18 hours. The separated crystalline material was filtered off, the filtrate concentrated and the residue chromatographed on a silica gel column (10 cm long) with hexane-ethyl acetate 4:1 as eluent to afford 58.9 g (91%).

¹H NMR (300 MHz, CDCl₃): δ 1.15 (t, 6H); 1.9-2.0 (m, 4H); 2.38 (s, 3H); 2.99 (m, 2H); 4.07 (q, 4H). ¹³C NMR (75 MHz, CDCl₃): δ 13.98; 27.68; 40.82; 60.39; 67.93; 68.06; 172.32.

### 3-Benzyl-8-methyl-3,8-diazabicyclo[3.2.1]octane-2,4-dione (Intermediate compound)

To a solution of diethyl *cis*-1-methylpyrrolidine-2,5-dicarboxylate (74.8 g; 0.383 mol) in xylene (150 ml) benzylamine (41.0 g; 0.383 mol) was added and the mixture heated to reflux for 16 hours. Then xylene was removed at reduced pressure and the residue was heated at 220°C for 18 hours. The obtained crude product was distilled by portions (30-40 g) on Büchi oven for distillation at 180°C and 0.1 mbar, and the first fraction collected (after about 1 hours). The combined first fractions were crystallized from a mixture of hexane and ethyl acetate 1:1 to yield 30.6 (34%).

¹H NMR (300 MHz, CDCl₃): δ 1.88 (m, 2H); 2.34 (m, 2H); 2.42 (s, 3H); 3.80 (dd, 2H); 4.88 (s, 2H); 7.2-7.4 (m, 5H). ¹³C NMR (75 MHz, CDCl₃):26.69; 35.82; 41.26; 65.72; 127.42; 128.36; 128.62; 136.91; 173.26.

### 3-Benzyl-8-methyl-3,8-diazabicyclo[3.2.1]octane (Intermediate compound)

To a solution of 3-benzyl-8-methyl-3,8-diazabicyclo[3.2.1]octane-2,4-dione (28.3 g; 0.116 mol) in 200 ml of absolute dioxane LiAlH₄ (7.6 g; 0.2 mol) was added and the mixture boiled under argon for 18 hours. Then a mixture of water (7.5 ml) and dioxane (40 ml) was added drop-wise to the reaction mixture. The suspension was mixed for 20 minutes and filtered trough a dense glass filter. The filtrate was evaporated and the residue was distilled on Büchi oven for distillation at 120°C and 0.1 mbar. Yield 17.6 g (70%).

¹H NMR (300 MHz, CDCl₃): δ 1.7-1.9 (m, 4H); 2.18 (s, 3H); 2.25 (d, 2H); 2.48 (dd, 2H); 2.95 (m, 2H); 3.39 (s, 2H); 7.1-7.3 (m, 5H).

### 8-Methyl-3,8-diazabicyclo[3.2.1]octane (Intermediate compound)

To a degassed by argon solution of 3-benzyl-8-methyl-3,8-diazabicyclo[3.2.1]octane (17.6 g; 0.08 mol) in methanol (50 ml), 10% Pd/C (1.0 g) was added and hydrogen passed into reaction mixture for 24 hours. The catalyst was filtered off, the filtrate evaporated and the residue distilled on Büchi oven for distillation at 100°C and 0.1 mbar. Yield 8.5 g (85%).

¹H NMR (300 MHz, CDCl₃): δ 1.6 (m, 2H); 1.86 (s, 1H); 1.9-2,0 (m, 2H); 2,17 (s, 3H); 2.53 (m, 2H); 2.9-3.0 (m, 4H). ¹³C NMR (75 MHz, CDCl₃): δ 24.73; 41.72; 52.10; 62.08.

### Method A

### 3-(6-Bromo-pyridazin-3-yl)-8-methyl-3,8-diaza-bicyclo[3.2.1]octane fumaric acid salt (Intermediate compound)

A mixture of 8-methyl-3,8-diazabicyclo[3.2.1]octane (2.0 g; 15.85 mmol), 3,6-dibromopyridazine (3.77 g; 15.85 mmol) and dioxane (20 ml) was stirred at room temperature for 15 hours. Aqueous sodium hydroxide (1 M; 20 ml) was added and the mixture was extracted twice with dichloromethane (2 x 20 ml). Chromatography on silica gel with dichloromethane, 10% methanol and 1% aqueous ammonia as solvent gave the compound as free base. Yield 1.83 g (41%). The corresponding salt was obtained by addition of a diethyl ether and methanol mixture (9:1) saturated with fumaric acid. Mp. 118.5-119.5°C.

### (±)-3-(6-Bromo-pyridazin-3-yl)-9-methyl-3,9-diaza-bicyclo[4.2.1]nonane fumaric acid salt (Intermediate compound)

Was prepared according to Method A from (±)-9-methyl-3,9-diazabicyclo-[4.2.1]-nonane. (±)-9-Methyl-3,9-diazabicyclo-[4.2.1]-nonane was prepared according to [Michaels RJ and Zaugg HE; J. Org. Chem. 1960 25 637]. Mp. 175.6°C.

### Method B

### 8-Methyl-3-(6-phenylethynyl-pyridazin-3-yl)-3,8-diaza-bicyclo[3.2.1]octane fumaric acid salt (Compound B1)

A mixture of 3-(6-bromo-pyridazin-3-yl)-8-methyl-3,8-diaza-bicyclo-[3.2.1]octane (1.63 g; 5.76 mmol), phenylacetylene (2.94 g; 28.8 mmol), palladacycle (109 mg; 0.115 mmol), Cul (1.09 g; 5.75 mmol), KI (955 mg; 5.76 mmol), diisopropylethylamine (1.49 g; 11.5 mmol), diehylamine (842 mg; 11.5 mmol) and dioxane (40 ml) was stirred at reflux for 15 hours. Aqueous sodium hydroxide (1 M; 20 ml) was added and the mixture was extracted twice with dichloromethane (2 x 20 ml). Chromatography on silica gel with dichloromethane, 10% methanol and 1% aqueous ammonia as solvent gave the compound as free base. Yield 1.2 g (68%). The corresponding salt was obtained by addition of a diethyl ether and methanol mixture (9:1) saturated with fumaric acid. Mp. 142.4°C.

### N-{4-[6-(8-Methyl-3,8-diaza-bicyclo[3.2.1]oct-3-yl)-pyridazin-3-ylethynyl]-phenyl}-acetamide (Compound B2)

Was prepared according to Method B from 3-(6-bromo-pyridazin-3-yl)-8-methyl-3,8-diaza-bicyclo[3.2.1]octane and N-(4-ethynyl-phenyl)-acetamide. Mp 292°C.

### Diethyl meso-2,6-dibromopimeloate (Intermediate compound 1)

Pimelic acid (240 g, 1.5 mol) was placed into a two-necked round bottom flask (1000 ml) fitted with a reflux condenser and an argon inlet. The reflux condenser was connected with two consecutive flasks (500 and 1000 ml). The first flask (500 ml) was placed in to dry ice-isopropanol vessel and the second was half filled with water for HCl absorption. Thionyl chloride (368 g, 3.09 mol) was added in three portions (180, 100 and 88 g) and stirred at 40°C until gas elution ceased. Finally temperature was raised to 100°C, the first flask with liquid SO₂ was disconnected. The flask was fitted with dropping funnel and gas outlet. During 3 hours the flask was continuously irradiated with 300 W UV lamp and bromine (490 g, 3.06 mol) was added dropwise. The HBr formed was absorbed in two consecutive water filled flasks (2 x 1000 ml). When HBr elution ceased, the dropping funnel was filled with absolute ethanol (200 ml) and carefully added dropwise. The chilled solution was washed with water, aqueous sodium acetate and sodium thiosulfate. The separated organic phase was dried over sodium sulfate, filtrated and distilled in multiple portions (about 40 ml each) by a Büchi oven *in vacuo* (0.5-1.0 mbar) at 150°C collecting the fraction from the third flask. Yield: 487 g (87%).

### Diethyl cis-1-Methylpiperidine-2,6-dicarboxylate (Intermediate compound 2)

Diethyl *meso*-2,6-dibromoadipoate **(1)** (236 g, 0.631 mol) was placed into a two necked round bottom flask (2000 ml) fitted with a reflux condenser and a thermometer, and was dissolved in absolute THF (400 ml) under argon. A solution of methylamine (62 g, 2.0 mol) in absolute THF (400 ml) was added to the solution of compound **1.** The flask was placed in cold water, to prevent it from warming. The reaction mixture was stirred for 18 hours under argon, the separated *N-*methylammonium bromide was removed by filtration and washed thoroughly with THF. The filtrate was concentrated on a rotary evaporator under reduced pressure and the residue (156 g) was distilled in four portions (about 39 g each) by a Büchi oven *in vacuo* (0.1-0.4 mbar) at 125°C (average distillation time 1 hour) collecting the fraction from the third flask. Yield of compound **2** 127.5 g (83%) as a light-yellowish oil.

### 3-Benzyl-9-methyl-3,9-diazabicyclo[3.3.1]nonane-2,4-dione (Intermediate compound 3)

A solution of diethyl *cis*-1-methylpiperidin-2,6-carboxylate (127.5 g, 0.524 mol) and benzylamine (57.8 g, 0.540 mol) in xylene (150 ml) was refluxed in a roundbottomed flask (250 ml) for 44 hours. The latter was equipped with a vertical air condenser (15 cm) followed by a Liebig condenser, allowing removal of ethanol from the reaction mixture. The xylene was removed under reduced pressure through a Liebig condenser, the oil bath temperature was elevated to 205°C and the mixture was heated under argon for 20 hours. The obtained product was distilled in four portions (about 45 g each) by a Büchi oven *in vacuo* (0.1 mbar) at 160°C (average distillation time 1 hour) collecting the fraction from the third flask. The three combined 3^{rd} fractions (96 g) were dissolved by boiling in 50 ml of ethyl acetate and allowed to crystallize at room temperature for 3 days. The crystalline material was filtered off, washed with a small amount of ethyl acetate and dried *in vacuo* to afford 39.5 g of the product as a white crystalline solid. The filtrate was concentrated and the residue crystallized from ethyl acetate (30 ml) at 4°C for 2 days to yield 6.2 g of the same product. Yield of compound 3 was 45.7 g (34%), mp. 117-118°C.

### 3-Benzyl-9-methyl-3,9-diazabicyclo[3.3.1]nonane (Intermediate compound 4)

To a solution of compound **3** (45.7 g, 0.177 mol) in 1,4-dioxane (400 ml) placed into a three-necked round bottom flask (1000 ml), LiAlH₄ (9.0 g, 0.237 mol) was added in small portions and the mixture was refluxed under argon for 18 hours. The reaction mixture was cooled to 80°C and a mixture of water (9 ml) and 1,4-dioxane (40 ml) was dropped carefully into reaction flask (caution: vigorous hydrogen evolution). A fine suspension was cooled to room temperature and treated with KOH solution (20 g in 50 ml of water). The organic phase was decanted and concentrated was concentrated under reduced pressure. The residue was distilled on Büchi oven *in vacuo* (0.1 mbar) at 130°C. The third collecting flask contained 3,9-diazabicyclo[3.3.1]nonane **4** (29.2 g, 72%) as a viscous colourless oil.

### 9-Methyl-3,9-diazabicyclo[3.3.1]nonane (Intermediate compound 5)

To a solution of compound **4** (28.7 g, 0.125 mol) in absolute ethanol (100 ml) was added 10% Pd/C catalyst (6.3 g) under argon. The solution was hydrogenated with H₂ at 60 bar and 100°C for 16 hours. The solution was filtered of on a Büchner funnel, the filtrate was concentrated under reduced pressure on a rotary evaporator and the residue distilled on Büchi oven *in vacuo* (0.1 mbar) at 100°C to afford compound 5 (8.5 g, 49%) as a colorless gel.

Reference related to the preparation of intermediate compounds 1-5: II Farmaco 55 (8) August 2000, Pages 553-562.

### Method C

### 3-(6-lodo-pyridazin-3-yl)-9-methyl-9-aza-bicyclo[3.3.1]nonane free base (Intermediate compound; JBP 18097-a)

A mixture of 9-methyl-3,9-diazabicyclo[3.3.1]nonane (4.0 g, 28.5 mmol), 3,6-diiodopyridazine (9.5 g, 28.5 mmol), diisopropylethylamine (7.4 g, 57.0 mmol) and dioxane (50 ml) was stirred at 75°C for 4 days. Aqueous sodium hydroxide (75 ml, 1 M) was added, dioxane was evaporated and the mixture was extracted twice with dichloromethane (2 x 75 ml). Chromatography on silica gel with dichloromethane, 10% methanol and 1% aqueous ammonia as solvent gave the title compound. Yield 4.61 g (47%). Mp. 163-166°C.

### Method D

### 9-Methyl-3-(6-phenylethynyl-pyridazin-3-yl)-9-aza-bicyclo[3.3.1]nonane fumaric acid salt (Compound D1)

3-(6-Iodo-pyridazin-3-yl)-9-methyl-9-aza-bicyclo[3.3.1]nonane (0.50 g, 1.45 mmol), phenylacetylene (0.30 g, 2.90 mmol), diisopropylethylamine (0.37 g, 2.90 mmol), Cul (28 mg, 0.14 mmol), palladacycle (27 mg, 0.029 mmol) and dioxane (20 ml) was stirred at reflux for 3 days. Aqueous sodium hydroxide (30 ml, 1 M) was added, dioxane was evaporated and the mixture was extracted twice with dichloromethane (2 x 30 ml). Chromatography on silica gel with dichloromethane, 10% methanol and 1% aqueous ammonia as solvent gave the title compound. Yield 0.38 g (82%). The corresponding salt was obtained by addition of a diethyl ether and methanol mixture (9:1) saturated with fumaric acid. Mp. 196-204°C.

### 5-Ethynyl-1H-indole (Intermediate compound)

A mixture of 4-(1*H*-indol-5-yl)-2-methyl-but-3-yn-2-ol (6.6 g, 33.1 mmol), sodiumhydride, 60% in mineral oil (0.13 g, 3.3 mmol) and anhydrous toluene (100 ml) was stirred for 5 hours at 110°C. The crude mixture was evaporated. Chromatography on silica gel with dichloromethane, 10% methanol and 1% aqueous ammonia as solvent gave the title compound. Yield 3.44 g (74%). Oil.

### 4-(1H-Indol-5-yl)-2-methyl-but-3-yn-2-ol (Intermediate compound)

A mixture of 5-iodoindole (25 g, 102.9 mmol), triphenylphosphine (2.70 g, 10.3 mmol), Cul (1.96 g, 10.3 mmol), potassium carbonate (35.5 g, 257.2 mmol), palladium (5 wt. % on calcium carbonate, poisoned with lead) (0.55 g, 5.14 mmol) and 1,2-dimethoxyethane (250 ml) was stirred at room temperature for 0.5 hour. 2-Methyl-3-butyn-2-ol (21.6 g, 257 mmol) was added and the mixture was stirred for 15 hours at 100°C. The crude mixture was filtered through silica gel and aqueous hydrochloric acid (250 ml, 2 M) was added and extracted with toluene. The organic phase was dried and evaporated. Chromatography on silica gel with dichloromethane, 10% methanol and 1% aqueous ammonia as solvent gave the title compound. Yield 6.7 g (33%). Mp. 119-123°C.

### Method E

### 4-[4-(9-Methyl-3,9-diaza-bicyclo[3.3.1]non-3-yl)-phenylethynyl]-phenylamine free base (Compound E1)

A mixture of *N*-{4-[6-(9-methyl-9-aza-bicyclo[3.3.1]non-3-yl)-pyridazin-3-ylethynyl]-phenyl}-acetamide (0.42 g, 1.11 mmol), aqueous sodium hydroxide (10 ml, 4 M) and ethanol (10 ml, 96%) was stirred for 2 hours at reflux. The mixture was evaporated. Water (30 ml) was added and the mixture was extracted twice with dichloromethane (2 x 30 ml). Chromatography on silica gel with dichloromethane, 10% methanol and 1% aqueous ammonia as solvent gave the title compound. Yield 0.30 g (80%). Mp.191 °C.

### 4-[6-(8-Methyl-3,8-diaza-bicyclo[3.2.1]oct-3-yl)-pyridazin-3-ylethynyl]-phenylamine free base (Compound E2)

Was prepared according to Method E from N-{4-[6-(8-methyl-3,8-diaza-bicyclo[3.2.1]oct-3-yl)-pyridazin-3-ylethynyl]-phenyl}-acetamide. Mp. 204-210°C.

### Method F

### N-{4-[6-(8-Methyl-3,8-diaza-bicyclo[3.2.1]oct-3-yl)-pyridazin-3-ylethynyl]-phenyl}-benzamide free base (Compound F1)

To a stirred mixture of 4-[6-(8-methyl-3,8-diaza-bicyclo[3.2.1]oct-3-yl)-pyridazin-3-ylethynyl]-phenylamine (0.53 g, 1.66 mmol) and dichloromethane (20 ml) was added: benzoylchloride (0.35 g, 2.5 mmol) solved in dichloromethane (20 ml). The mixture was stirred at 0°C for 2 hours and was then allowed to stir at room temperature for 15 hours. Aqueous sodium hydroxide (20 ml, 1 M) was added and the mixture was extracted with dichloromethane (3 X 30 ml). The mixture was dried and evaporated. Mp 88-96°C. LC-ESI-HRMS of [M+H]+ shows 424.2145 Da. Calc. 424.213735 Da, dev. 1.8 ppm.

### N-{4-[6-(8-Methyl-3,8-diaza-bicyclo[3.2.1]oct-3-yl)-pyridazin-3-ylethynyl]-phenyl}-propionamide fumaric acid salt (Compound F2)

Was prepared according to Method F from 4-[6-(8-methyl-3,8-diaza-bicyclo[3.2.1]oct-3-yl)-pyridazin-3-ylethynyl]-phenylamine and propionic anhydride. Mp 238°C. LC-ESI-HRMS of [M+H]+ shows 376.2153 Da. Calc. 376.213735 Da, dev. 4.2 ppm.

### N-{4-[6-(8-Methyl-3,8-diaza-bicyclo[3.2.1]oct-3-yl)-pyridazin-3-ylethynyl]-phenyl}-isobutyramide fumaric acid salt (Compound F3)

Was prepared according to Method F from 4-[6-(8-methyl-3,8-diaza-bicyclo[3.2.1]oct-3-yl)-pyridazin-3-ylethynyl]-phenylamine and isobutyric anhydride. Mp 214°C. LC-ESI-HRMS of [M+H]+ shows 390.231 Da. Calc. 390.229385 Da, dev. 4.1 ppm.

### N-(4-Ethynyl-phenyl)-acetamide (Intermediate compound)

Was prepared according to Method F from 4-ethynyl-phenylamine.

### 8,8-Dimethyl-3-(6-phenylethynyl-pyridazin-3-yl)-3,8-diaza-bicyclo[3.2.1]octane onium iodide salt (Compound F5)

Iodomethane (0.23 g, 1.6 mmol) solved in dichloromethane (20 ml) was added to mixture of 8-methyl-3-(6-phenylethynyl-pyridazin-3-yl)-3,8-diaza-bicyclo[3.2.1]octane (0.50 g, 1.6 mmol) and dichloromethane (20 ml) at -70°C. The mixture was stirred at -70°C for 1 hour. The mixture was allowed to reach room temperature and was stirred for 3 days. The mixture was evaporated and a mixture of methanol/ether (5%, 20 ml) was added, followed by filtration. The crystalline product was dried. LC-ESI-HRMS of M+ shows 319.1935 Da. Calc. 319.1923 Da, dev. 3.8 ppm.

### Example 2

### In vitro Inhibition of ³H-α-Bungarotoxine Binding in Rat Brain

In this example the affinity of the compounds of the invention for binding to α₇-subtype of nicotinic receptors is determined.

α-Bungarotoxine is a peptide isolated from the venom of the Elapidae snake *Bungarus multicinctus.* It has high affinity for neuronal and neuromuscular nicotinic receptors, where it acts as a potent antagonist. ³H-α-Bungarotoxine labels nicotinic acetylcholine receptors formed by the α₇ subunit isoform found in brain and the α₁ isoform in the neuromuscular junction.

### Tissue preparation

Preparations are performed at 0-4°C. Cerebral cortices from male Wistar rats (150-250 g) are homogenised for 10 seconds in 15 ml of 20 mM Hepes buffer containing 118 mM NaCl, 4.8 mM KCl, 1.2 mM MgSO₄ and 2.5 mM CaCl₂ (pH 7.5) using an Ultra-Turrax homogeniser. The tissue suspension is subjected to centrifugation at 27,000 x g for 10 minutes. The supernatant is discarded and the pellet is washed twice by centrifugation at 27,000 x g for 10 minutes in 20 ml of fresh buffer, and the final pellet is then re-suspended in fresh buffer containing 0.01% BSA (35 ml per g of original tissue) and used for binding assays.

### Assay

Aliquots of 500 µl of homogenate are added to 25 µl of test solution and 25 µl of ³H-α-bungarotoxine (2 nM, final concentration) and mixed and incubated for 2 hours at 37°C. Non-specific binding is determined using (-)-nicotine (1 mM, final concentration). After incubation, the samples are added 5 ml of ice-cold Hepes buffer containing 0.05% PEI and poured directly onto Whatman GF/C glass fibre filters (pre-soaked in 0.1% PEI for at least 6 hours) under suction, and immediately washed with 2 x 5 ml ice-cold buffer.

The amount of radioactivity on the filters is determined by conventional liquid scintillation counting. Specific binding is total binding minus non-specific binding.

The test value is given as an IC₅₀ (the concentration of the test substance which inhibits the specific binding of ³H-α-bungarotoxin by 50%).

The results of these experiments are presented in Table 1 below.

**Table 1**

| Inhibition of ³H-α-Bungarotoxine Binding | |
|---|---|
| **Compound No.** | **IC₅₀ (**µ**M)** |
| B1 | 0.47 |

## Claims

1. A diazabicyclic aryl derivative represented by Formula I 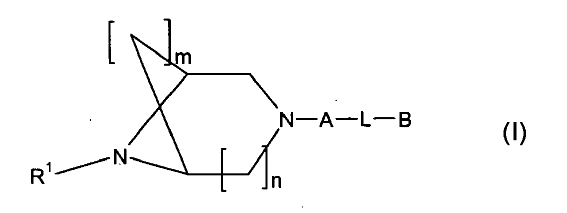 any of its enantiomers or any mixture of its enantiomers, or a pharmaceutically acceptable salt thereof, wherein
m is 2; and n is 1;
R¹ represents hydrogen or C₁₋₆-alkyl;
A represents a pyridazinyl group;
B represents a phenyl group, which phenyl may optionally be substituted with amino or -NH(CO)R'; wherein R' represents C₁₋₆-alkyl; and
L represents -C≡C-.

2. The diazabicyclic aryl derivative of claim 1, which is
8-Methyl-3-(6-phenylethynyl-pyridazin-3-yl)-3,8-diaza-bicyclo[3.2.1]octane;
N-{4-[6-(8-Methyl-3,8-diaza-bicyclo[3.2.1]oct-3-yl)-pyridazin-3-ylethynyl]-phenyl}-acetamide;
4-[6-(8-Methyl-3,8-diaza-bicyclo[3.2.1]oct-3-yl)-pyridazin-3-ylethynyl]-phenylamine;
N-{4-[6-(8-Methyl-3,8-diaza-bicyclo[3.2.1]oct-3-yl)-pyridazin-3-ylethynyl]-phenyl}-propionamide;
N-{4-[6-(8-Methyl-3,8-diaza-bicyclo[3.2.1]oct-3-yl)-pyridazin-3-ylethynyl]-phenyl}-isobutyramide; or
8,8-Dimethyl-3-(6-phenylethynyl-pyridazin-3-yl)-3,8-diaza-bicyclo[3.2.1]octane onium iodide salt;
or an enantiomers or a mixture of its enantiomers, or a pharmaceutically acceptable salt thereof.

3. A pharmaceutical composition comprising a therapeutically effective amount of the diazabicyclic aryl derivative of either one of claims 1-2, or a pharmaceutically acceptable addition salt thereof, together with at least one pharmaceutically acceptable carrier or diluent.

4. Use of the diazabicyclic aryl derivative of either one of claims 1-2, or a pharmaceutically acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

5. The use according to claim 4, wherein the disease, disorder or condition is a cognitive disorder, learning deficit, memory deficits and dysfunction, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, Amyotrophic Lateral Sclerosis, anxiety, non-0CD anxiety disorders, convulsive disorders, epilepsy, neurodegenerative disorders, transient anoxia, induced neuro-degeneration, neuropathy, diabetic neuropathy, periferic dyslexia, tardive dyskinesia, hyperkinesia, mild, pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to postherpetic neuralgia, or to peripheral nerve injury, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, or withdrawal symptoms caused by termination of use of addictive substances, including nicotine containing products such as tobacco, opioids such as heroin, cocaine and morphine, benzodiazepines and benzodiazepine-like drugs, and alcohol.

## Patentansprüche

1. Diazabicyclisches Arylderivat, das durch Formel I wiedergegeben ist 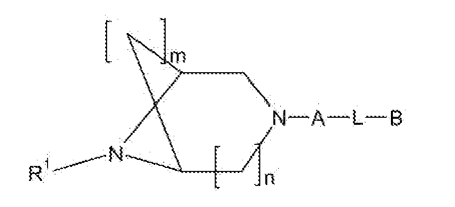 beliebige von seinen Enantiomeren oder eine beliebige Mischung von seinen Enantiomeren, oder ein pharmazeutisch verträgliches Salz davon, wobei
m 2 ist; und n 1 ist;
R¹ Wasserstoff oder C₁₋₆-Alkyl bedeutet;
A eine Pyridazinylgruppe bedeutet;
B eine Phenylgruppe bedeutet, wobei dieses Phenyl gegebenenfalls mit Amino oder -NH(CO)R' substituiert sein kann; wobei R' C₁₋₆-Alkyl bedeutet; und
L -C≡C- bedeutet.

2. Diazabicyclisches Arylderivat nach Anspruch 1, welches
8-Methyl-3-(6-phenylethinyl-pyridazin-3-yl)-3,8-diaza-bicyclo[3.2.1]octan;
N-{4-[6-(8-Methyl-3,8-diaza-bicyclo[3.2.1]oct-3-yl)-pyridazin-3-ylethinyl]-phenyl}-acetamid;
4-[6-(8-Methyl-3,8-diaza-bicyclo[3.2.1]oct-3-yl)-pyridazin-3-ylethinyl]-phenylamin;
N-{4-[6-(8-Methyl-3,8-diaza-bicyclo[3.2.1]oct-3-yl)-pyridazin-3-ylethinyl]-phenyl}-propionamid;
N-{4-[6-(8-Methyl-3,8-diaza-bicyclo[3.2.1]oct-3-yl)-pyridazin-3-ylethinyl]-phenyl}-isobutyramid; oder
8,8-Dimethyl-3-(6-phenylethinyl-pyridazin-3-yl)-3,8-diaza-bicyclo[3.2.1]octan-onium-iodid-salz ist;
oder ein Enantiomer bzw. Enantiomere oder eine Mischung von seinen Enantiomeren, oder ein pharmazeutisch verträgliches Salz davon.

3. Pharmazeutische Zusammensetzung umfassend eine therapeutisch wirksame Menge des diazabicyclischen Arylderivats nach einem der Ansprüche 1-2, oder eines pharmazeutisch verträglichen Additionssalzes davon, zusammen mit wenigstens einem pharmazeutisch verträglichen Träger oder Verdünnungsmittel.

4. Verwendung des diazabicyclischen Arylderivats nach einem der Ansprüche 1-2, oder eines pharmazeutisch verträglichen Additionssalzes davon, für die Herstellung einer pharmazeutischen Zusammensetzung/eines Medikaments für die Behandlung, Verhütung oder Linderung einer Krankheit oder einer Störung oder eines Zustands eines Säugers, einschließlich eines Menschen, wobei die Krankheit, die Störung oder der Zustand auf die Modulation von cholinergen Rezeptoren und/oder Monoaminrezeptoren anspricht.

5. Verwendung nach Anspruch 4, wobei es sich bei der Krankheit, der Störung oder dem Zustand um eine kognitive Störung, ein Lerndefizit, Gedächtnisdefizite und eine Gedächtnisdysfunktion, die Alzheimer-Krankheit, ein Aufmerksamkeitsdefizit, eine Aufmerksamkeitsdefizit-Hyperaktivitätsstörung (ADHD), das Tourette-Syndrom, eine Psychose, eine Depression, eine bipolare Störung, eine Manie, eine manische Depression, eine Schizophrenie, kognitive Defizite oder Aufmerksamkeitsdefizite in Verbindung mit Schizophrenie, Zwangsstörungen (OCD), Panikstörungen, Essstörungen wie Anorexia nervosa, Bulimie und Fettleibigkeit, Narkolepsie, Nozizeption, AIDS-Demenz, senile Demenz, Autismus, die Parkinson-Krankheit, Chorea Huntington, amyotrophe Lateralsklerose, Angst, Nicht-OCD-Angststörungen, konvulsive Störungen, Epilepsie, neurodegenerative Störungen, vorübergehende Anoxie, induzierte Neurodegeneration, Neuropathie, diabetische Neuropathie, periphere Dyslexie, tardive Dyskinesie, Hyperkinesie, milde Schmerzen, mäßige oder starke Schmerzen, Schmerzen von akutem, chronischem oder wiederkehrendem Charakter, durch Migräne verursachte Schmerzen, postoperative Schmerzen, Phantomschmerzen, entzündliche Schmerzen, neuropathische Schmerzen, chronische Kopfschmerzen, zentrale Schmerzen, Schmerzen in Verbindung mit diabetischer Neuropathie, mit postherpetischer Neuralgie oder mit peripherer Nervenverletzung, Bulimie, das posttraumatische Syndrom, eine Sozialphobie, Schlafstörungen, eine Pseudodemenz, das Ganser-Syndrom, das prämenstruelle Syndrom, das Syndrom der späten Lutealphase, das chronische Ermüdungssyndrom, Mutismus, Trichotillomanie, Jetlag, Arrhythmien, Kontraktionen eines glatten Muskels, Angina pectoris, eine Frühgeburt, eine Diarrhö, Asthma, tardive Dyskinesie, Hyperkinesie, eine vorzeitige Ejakulation, Erektionsschwierigkeiten, Hochdruck, entzündliche Störungen, entzündliche Hautstörungen, Akne, Rosacea, Morbus Crohn, eine entzündliche Darmerkrankung, Colitis ulcerosa, eine Diarrhö oder Entzugssymptome, die durch die Beendigung des Gebrauchs von süchtigmachenden Substanzen, einschließlich nikotinhaltiger Produkte wie Tabak, Opioide wie Heroin, Kokain und Morphin, Benzodiazepine und benzodiazepinartiger Drogen bzw. Arzneimittel, und Alkohol, verursacht sind, handelt.

## Revendications

1. Dérivé arylé diazabicyclique représenté par la formule I 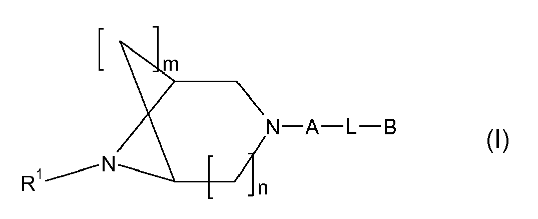 l'un quelconque de ses énantiomères ou tout mélange de ses énantiomères, ou un sel pharmaceutiquement acceptable de celui-ci,
dans laquelle
m est 2 ; et n est 1 ;
R¹ représente un hydrogène ou un alkyle en C₁₋₆ ;
A représente un groupe pyridazinyle ;
B représente un groupe phényle, lequel phényle peut être facultativement substitué par un amino ou -NH(CO)R' ; où R' représente un alkyle en C₁₋₆ ; et
L représente -C≡C-.

2. Dérivé arylé diazabicyclique selon la revendication 1, qui est
le 8-méthyl-3-(6-phényléthynyl-pyridazin-3-yl)-3,8-diaza-bicyclo[3.2.1]octane ;
le N-{4-[6-(8-méthyl-3,8-diaza-bicyclo[3.2.1]oct-3-yl)-pyridazin-3-yléthynyl]-phényl}-acétamide ;
la 4-[6-(8-méthyl-3,8-diaza-bicyclo[3.2.1]oct-3-yl)-pyridazin-3-yléthynyl]-phénylamine ;
le N-{4-[6-(8-méthyl-3,8-diaza-bicyclo[3.2.1]oct-3-yl)-pyridazin-3-yléthynyl]-phényl}-propionamide ;
le N-{4-[6-(8-méthyl-3,8-diaza-bicyclo[3.2.1]oct-3-yl)-pyridazin-3-yléthynyl]-phényl}-isobutyramide ; ou
le sel iodure d'onium de 8,8-diméthyl-3-(6-phényléthynyl-pyridazin-3-yl)-3,8-diaza-bicyclo[3.2.1]octane ;
ou un énantiomère ou un mélange de ses énantiomères, ou un sel pharmaceutiquement acceptable de celui-ci.

3. Composition pharmaceutique comprenant une quantité thérapeutiquement efficace du dérivé arylé diazabicyclique selon l'une quelconque des revendications 1-2, ou d'un sel d'addition pharmaceutiquement acceptable de celui-ci, conjointement avec au moins un support ou diluant pharmaceutiquement acceptable.

4. Utilisation du dérivé arylé diazabicyclique selon l'une quelconque des revendications 1-2, ou d'un sel d'addition pharmaceutiquement acceptable de celui-ci, pour la fabrication d'une composition pharmaceutique/médicament pour le traitement, la prévention ou l'atténuation d'une maladie ou d'un trouble ou d'un état d'un mammifère, incluant un être humain, laquelle maladie, lequel trouble ou état réagit à une modulation des récepteurs cholinergiques et/ou des récepteurs monoamines.

5. Utilisation selon la revendication 4, dans laquelle la maladie, le trouble ou l'état est un trouble cognitif, un défaut d'apprentissage, des déficiences et un dysfonctionnement de la mémoire, une maladie d'Alzheimer, une déficience de l'attention, un trouble d'hyperactivité avec déficience de l'attention (THADA), un syndrome de Tourette, une psychose, une dépression, un trouble bipolaire, une manie, une manie dépression, une schizophrénie, des déficiences cognitives ou de l'attention liées à une schizophrénie, des troubles obsessivo-compulsifs (TOC), des troubles paniques, des troubles de l'alimentation tels qu'une anorexie mentale, une boulimie et une obésité, une narcolepsie, une nociception, une démence liée au SIDA, une démence sénile, un autisme, une maladie de Parkinson, une maladie de Huntington, une sclérose latérale amyotrophique, une anxiété, des troubles d'anxiété non TOC, des troubles convulsifs, une épilepsie, des troubles neurodégénératifs, une anoxie transitoire, une neurodégénérescence induite, une neuropathie, une neuropathie diabétique, une dyslexie périphérique, une dyskinésie tardive, une hyperkinésie, une douleur légère, une douleur modérée ou sévère, une douleur de caractère aigu, chronique ou récurrent, une douleur provoquée par une migraine, une douleur postopératoire, une douleur de membre fantôme, une douleur inflammatoire, une douleur neuropathique, une céphalée chronique, une douleur centrale, une douleur liée à une neuropathie diabétique, à une névralgie post-zostérienne, ou à une lésion de nerf périphérique, une boulimie, un syndrome post-traumatique, une phobie sociale, des troubles du sommeil, une pseudodémence, un syndrome de Ganser, un syndrome prémenstruel, un syndrome de phase lutéale tardive, un syndrome de fatigue chronique, un mutisme, une trichotillomanie, un décalage horaire, des arythmies, des contractions des muscles lisses, une angine de poitrine, un travail prématuré, une diarrhée, un asthme, une dyskinésie tardive, une hyperkinésie, une éjaculation prématurée, une difficulté d'érection, une hypertension, des troubles inflammatoires, des troubles cutanés inflammatoires, une acné, une rosacée, une maladie de Crohn, une maladie intestinale inflammatoire, une recto-colite hémorragique, une diarrhée, ou des symptômes de sevrage provoqués par l'arrêt de l'utilisation de substances toxicomanogènes, incluant les produits contenant de la nicotine tel que le tabac, les opioïdes tels que l'héroïne, la cocaïne et la morphine, les benzodiazépines et les substances médicamenteuses de type benzodiazépine, et l'alcool.
